# EUROPÄISCHE PATENTSCHRIFT

(11) **EP 0 922 502 B1**
(45) Veröffentlichungstag und Bekanntmachung des Hinweises auf die Patenterteilung: **11.08.2004**
(21) Anmeldenummer: 98122136.9
(22) Anmeldetag: 25.11.1998
(51) Int. Cl.: B05D 5/10, B05D 5/08, B05D 7/04, B05C 1/10, B05C 5/02, A61F 13/00, A61F 13/02

(54) **Verfahren zur zumindest partiellen direkten Beschichtung eines dehnfähigen Trägermaterials mit einer Haftklebemasse**
Process for direct coating at least partially a stretchable substrate with an adhesive composition
Procédé pour revêtir en mode direct au moins partiellement un substrat étirable avec une composition adhésive

(30) Priorität: 13.12.1997 DE 19755437
(43) Veröffentlichungstag der Anmeldung: 16.06.1999
(73) Patentinhaber: Beiersdorf Aktiengesellschaft, 20245 Hamburg (DE)
(72) Erfinder: Himmelsbach, Peter, 21614 Buxtehude (DE); Jauchen, Peter, 22455 Hamburg (DE); Keite-Telgenbüscher, Klaus, Dr., 22529 Hamburg (DE); Lehder, Matthias, 21244 Buchholz (DE)

(56) Entgegenhaltungen:
- EP-A- 0 922 503
- WO-A-97/43993
- DE-A- 3 346 100
- DE-A- 19 532 387
- US-A- 3 102 046

## Beschreibung

Die Erfindung betrifft ein Verfahren zur zumindest partiellen direkten Beschichtung eines dehnfähigen Trägermaterials mit einer Haftklebemasse, wobei das Trägermaterial mittels einer transportierenden Vorrichtung derartig gegen eine Beschichtungsvorrichtung geführt wird, daß durch die Beschichtungsvorrichtung die Haftklebemasse auf das Trägermaterial aufgetragen wird.

Die Verwendung dehnfähiger Materialien als Träger, die elastische oder plastisch formbare Eigenschaften aufweisen, sind in der Technik und Medizin bekannt. Sie finden ihre Verwendung in den verschiedensten Bereichen, u.a. auch als Basismaterialien in der Pflaster- und Klebebindenherstellung

Unter dem Begriff "plastisch oder elastisch verformbar" soll die Dehnfähigkeit eines Materials verstanden werden. Ein Material ist danach dehnfähig, wenn es sich bei einer Belastung von 10N/cm einen Längenzuwachs von mindestens 20% aufweist.

Ferner ist bekannt, daß die dehnfähigen Trägermaterialien sich mit diversen Klebemassensystemen beschichten lassen. Die Beschichtung ist generell vollflächig oder aber auch partiell, d.h. teilweise, möglich. Für medikale selbstklebend ausgerüstete Trägermaterialien zeigt sich hierbei, daß sich bei entsprechend poröser Trägermaterialien ein stark luft- und wasserdampfdurchlässiger Film ergibt, der in der Regel nach dem Verkleben auf der Haut des Patienten auch leichter wieder abzulösen ist. Als Verfahren zur Herstellung solcher partiell beschichteten Trägermaterialien ist der Rasterdruck verbreitet, insbesondere der Sieb-, Gravur- oder Flexodruck. Weiter ist bekannt, daß, zum Beispiel bei der Verwendung als Fixierungen, auch die selbstklebende Ausrüstung auf mehr als einer Seite erfolgen kann.

Als Klebemassen sind grundsätzlich Systeme auf Lösemittel- oder Dispersionsbasis oder aber auch 100 %-Systeme verwendbar. Vorteilhaft ist bei der Verarbeitung der 100 %-Systeme, daß ein Entfernen der Löse- oder Dispergierhilfsmittel vermieden wird. Hierdurch steigert sich die Produktivität und gleichzeitig wird der Maschinen- und Energieaufwand reduziert.

Elastische oder plastisch formbare Trägermaterialien lassen sich generell direkt oder indirekt beschichten.
Bei der indirekten Beschichtung wird zunächst ein vorzugsweise starrer oder wenig elastischer Hilfsträger beschichtet und sodann die Klebemasse in einem Kaschierprozeß auf den elastischen oder plastisch formbaren Träger transferiert. Als vorteilhaft für die indirekte Beschichtung hat sich herausgestellt, daß sich das Trägermaterial hierbei im Wesen unverformt und unvorbelastet zukaschieren läßt, wodurch Form und textiltechnologische Eigenschaften wie Flächengewicht, Höchstzugkraft, Höchstzugkraft-Dehnung, Hysterese-Dehnung weitestgehend erhalten bleiben.
Der Nachteil der indirekten Beschichtung liegt in der relativ schlechten Verankerung der Klebemasse auf dem elastischen oder plastisch verformbaren Träger. Hier zeigt es sich, daß insbesondere bei temperatursensiblen Trägermaterialien eine Thermokaschierung oder Laminierung nicht möglich ist oder einen nur geringen Erfolg aufweist. Bei dicken, porösen Trägermaterialien kann es beim Kaschieren dazu kommen, daß die Klebeschicht vollkommen in den Träger hineingedrückt wird, so daß sich sowohl die klebetechnischen Eigenschaften wie auch das Elastizitätsverhalten drastisch verschlechtern.

Bei der Direktbeschichtung läßt sich zwar eine deutlich verbesserte Verankerung der Klebemasse einstellen, jedoch wird der Träger thermisch und mechanisch stärker beansprucht. Insbesondere beim partiellen Klebemassenauftrag zeigt sich bei zumindest teilweise elastisch oder plastisch formbarem Trägermaterial, daß dieses, obwohl der Träger unbelastet und unverformt in die Beschichtungseinheit gegeben wird, teilweise so beansprucht wird, daß sich seine Eigenschaften irreparabel ändern. Dieses kommt dadurch zustande, daß das Klebesystem mit einer systemabhängigen Kraft an der Beschichtungssystem haftet.

Um den selbstklebend ausgerüsteten Träger von dieser Beschichtungseinheit zu trennen, muß eine Kraft auf den Träger aufgebracht werden, wodurch dieser eine Verschlechterung in seinen Eigenschaften, insbesondere in der Elastizität und dem Flächengewicht, erfährt.

Aufgabe der Erfindung war es, ein direktes Beschichtungsverfahren zu entwickeln, welches es ermöglicht, ein dehnfähiges Trägermaterial wenigstens partiell mit einer Haftklebemasse zu beschichten, ohne die Trägereigenschaften zu verändern.

Gelöst wird die Aufgabe durch ein Verfahren, wie es im Anspruch 1 näher beschrieben ist. Die Unteransprüche stellen vorteilhafte Ausführungsformen dar.

Danach wird beim erfindungsgemäßen Verfahren zur zumindest partiellen direkten Beschichtung eines dehnfähigen Trägermaterials mit einer Haftklebemasse das Trägermaterial mittels einer transportierenden Vorrichtung derartig gegen eine Beschichtungsvorrichtung geführt wird, daß durch die Beschichtungsvorrichtung die Haftklebemasse auf das Trägermaterial aufgetragen wird, wobei die Beschichtungsvorrichtung abhäsiv ausgerüstet ist, so daß bei der Beschichtung die Eigenschaften des Trägers nicht verändert werden.

In einer vorteilhaften Ausführungsform des Verfahrens erfolgt die Beschichtung des Trägermaterials vollflächig.

Vorzugsweise besteht die abhäsive Ausrüstung aus Silikone oder Fluor enthaltende Verbindungen oder plasmabeschichteten Trennsystemen.

Weiter vorzugsweise ist der abhäsive Auftrag mit einem Flächengewicht von 0,01 g/m² bis 350 g/m² aufgetragen, bevorzugt 0,1 g/m² bis 10 g/m².

In einer besonders bevorzugten Ausführungsform bestehen die transportierende Vorrichtung aus einer Druckwalze und die Beschichtungsvorrichtung aus einer rotierenden, nahtlosen, trommelförmigen und perforierten sowie abhäsiv ausgerüsteten Rundschablone, die über eine Düse mit der Haftklebemasse beschickt wird, wobei die Haftklebemasse über eine Düsenlippe durch die Rundschablone auf das vorbeigeführte Trägermaterial aufgebracht wird.
Bevorzugt kann die Druckwalze aus Metall, Keramik oder Kunststoff bestehen. Sie kann generell plastisch, elastisch oder starr ausgebildet sein.

Weiterhin können auf der transportierenden Vorrichtung Haft- oder Halteeinrichtungen vorhanden sein.

Die Haltevorrichtungen bestehen insbesondere aus Nadeln, die aus der transportierenden Vorrichtung ragen und in das Trägermaterial eingreifen und die eine Länge aufweisen größer 10 µm, bevorzugt zwischen 30 µm und 5000 µm, besonders bevorzugt zwischen 35 µm und 1000 µm.

Darüber hinaus können die Nadeln zumindest teilweise auf der Oberfläche der transportierenden Vorrichtung beweglich sein.

Als sehr vorteilhaft hat sich die Kombination aus Thermosiebdruck mit Nadeln in der Gegendruckwalze erwiesen. Je nach Anwendungszweck ist es möglich, die Gegendruckwalze in ihrer Beschaffenheit so auszurüsten, daß die Rauhigkeit beziehungsweise die Nadeln gleichmäßig, zufällig verteilt oder in einem bestimmten geometrischen Muster auf der Walzenoberfläche vorliegen. An den Träger angepaßt werden auch die geometrische Form und Ausdehnung der Haftelemente. Als vorteilhaft hat sich auch die Ausgestaltung der Nadelorientierung gezeigt.
Der Winkel der Nadelorientierung kann für spezielle Verwendungen zwischen 10° und 170° zur Tangente an die Oberfläche der Walze in Beschichtungsrichtung und senkrecht zur Beschichtungsrichtung betragen. Des weiteren kann der Winkel der Nadelorientierung für spezielle Verwendungen zwischen 10° und 170° zur Bahnrichtung betragen.
Die Nadeln können zudem wenigstens teilweise beweglich ausgelegt werden, so daß ihre Orientierung und/oder Größe sich, zum Beispiel durch Magnetfeldeinwirkung oder Exzenterkonstruktionen, während einer Umdrehung der Gegendruckwalze ändern kann.

Die Oberfläche der Gegendruckwalze und/oder der Haftelemente kann weiter sowohl physikalisch als auch chemisch vorbehandelt werden. Beispielhaft seien hier das Silikonisieren und das Teflonisieren genannt. Eine statische oder aber auch eine antistatische Behandlung können anwendungsbezogen Vorteile zeigen.
Verfahren zum Aufbringen solcher Trennbeläge sind in der technischen Literatur ausreichend beschrieben, beispielhaft sind die Tauch-, Streich-, Sprüh-, und Druckbeschichtung genannt. Die Trennbeläge können sowohl physikalisch als auch chemisch ausgehärtet werden. Vorteilhaft haben sich chemisch aushärtende Systeme für die Verarbeitung von Heißschmelzklebemassen gezeigt.

Eine weitere vorteilhafte Ausführungsform der Haltevorrichtungen auf der transportierenden Vorrichtung besteht in der Verwendung sehr rauher Oberflächen, d.h. in einer im allgemeinen ungeordneten Anordnung von zum Eingreifen in den dehnfähigen Träger geeigneten Geometrien.
Die aufgerauhte Oberfläche kann aus aufgebrachten Hartstoffpartikeln und/oder aus einer durch das Formgebungsverfahren oder durch mechanische, physikalische oder chemische Behandlung aufgerauhten Metall-, Keramik- oder Kunststoffoberfläche geformt sein.
Beispielhaft seien hier Korund- oder ähnliche Hartstoffbeschichtungen erwähnt, die eine schmirgelpapierähnliche Oberfläche aufweisen. Aber auch durch Ätz- oder andere Verfahren aufgerauhte Metalloberflächen sind geeignet.

Die Rauhtiefe der Oberfläche liegt vorteilhafterweise zwischen 30 und 5000 µm.

Aber auch durch Adhäsionskräfte wirksame Haltevorrichtungen, zum Beispiel der Einsatz einer in ihrer Klebkraft auf das Gesamtsystem abgestimmten Selbstklebemasse, sind möglich.

Für geeignete Trägermaterialien bieten sich darüber hinaus elektromagnetische Felder zum Applizieren der Haltekräfte an.

Beispielhaft soll das Verfahren am Prinzip des Thermosiebdrucks beschrieben, ohne damit die Erfindung unnötig einschränken zu wollen.

Das Prinzip des Thermosiebdrucks besteht in der Verwendung einer rotierenden beheizten, nahtlosen, trommelförmigen perforierten Rundschablone, die über eine Düse mit der Haftklebemasse beschickt wird. Eine speziell geformte Düsenlippe (Rund- oder Vierkantrakel) preßt die über einen Kanal eingespeiste Selbstklebemasse durch die Perforation der Schablonenwand auf die vorbeigeführte Trägerbahn. Diese wird mit einer Geschwindigkeit, die der Umgangsgeschwindigkeit der rotierenden Siebtrommel entspricht, mittels einer Gegendruckwalze gegen den Außenmantel der beheizten Siebtrommel geführt.

Die Releaseausrüstung der mechanisch aufgerüsteten Gegendruckwalze kann mittels unterschiedlichster Verfahren geschehen. Gebräuchliche Antiadhäsivbeschichtungen verlaufen über Silikon- oder Fluorpolymer-Coatingausrüstungen. Die gebräuchlichsten Polymersysteme sind kondensations- oder additionsvernetzende Lösungen. Neue Verfahren wie das Plasmacoatingsystem ergänzen die Reihe der Möglichkeiten.

Diese Gegendruckwalze ist mit einer benadelten Oberfläche so ausgerüstet, daß sie eine Kraft ausüben kann, welche richtungsabhängig so orientiert ist, daß sie geringfügig größer als die Haftungskraft der erkaltenden Kleberschmelze an der Siebtrommeloberfläche ist.
Durch lotrechtes Anlüften wird die Trägerbahn von der Gegendruckwalze entfernt.

Die Ausbildung der Klebstoffkalotten bleibt von den o.a. Vorrichtungen unberührt und geschieht bei der Herstellung des partiell beschichteten elastischen oder plastisch verformbaren Trägermaterials nach folgendem Mechanismus:

Der Düsenrakeldruck fördert die Haftklebemasse durch die Siebperforation an das Trägermaterial. Die Größe der ausgebildeten Kalotten wird durch den Durchmesser des Siebloches vorgegeben. Entsprechend der Transportgeschwindigkeit der Trägerbahn (Rotationsgeschwindigkeit der Siebtrommel) wird das Sieb vom Träger abgehoben. Bedingt durch die hohe Adhäsion der Haftklebemasse und die innere Kohäsion des Hotmelts wird von der auf dem Träger bereits haftenden Basis der Kalotten der in den Löchern begrenzte Vorrat an Haftklebemasse konturenscharf abgezogen bzw. durch den Rakeldruck auf den Träger gefördert.
Nach Beendigung dieses Transportes formt sich, abhängig von der Rheologie der Haftklebemasse, über der vorgegebenen Basisfläche die mehr oder weniger stark gekrümmte Oberfläche der Kalotte. Das Verhältnis Höhe zur Basis der Kalotte hängt vom Verhältnis Lochdurchmesser zur Wandstärke der Siebtrommel und den physikalischen Eigenschaften (Fließverhalten, Oberflächenspannung und Benetzungswinkel auf dem Trägermaterial) der Selbstklebemasse ab.

Der beschriebene Bildungsmechanismus der Kalotten erfordert bevorzugt saugfähige oder zumindest von Haftklebemasse benetzbare Trägermaterialien.

Nichtbenetzende Trägeroberflächen müssen durch chemische oder physikalische Verfahren vorbehandelt werden. Dies kann durch zusätzliche Maßnahmen wie zum Beispiel Coronaentladung oder Beschichtung mit die Benetzung verbessernden Stoffen geschehen.

Mit dem aufgezeigten Druckverfahren kann die Größe und Form der Kalotten definiert festgelegt werden. Die für die Anwendung relevanten Klebkraftwerte, die die Qualität der erzeugten Produkte bestimmen, liegen bei sachgerechter Beschichtung in sehr engen Toleranzen. Der Basisdurchmesser der Kalotten kann von 10 µm bis 5000 µm gewählt werden, die Höhe der Kalotten von 20 µm bis ca. 2000 µm, bevorzugt 50 µm bis 1000 µm, wobei der Bereich kleiner Durchmesser für glatte Träger, der mit größerem Durchmesser und größerer Kalottenhöhe für rauhe oder stark porige Trägermaterialien vorgesehen ist.
Die Positionierung der Kalotten auf dem Träger wird durch die in weiten Grenzen variierbare Geometrie des Auftragswerkes, z.B. Gravur- oder Siebgeometrie, definiert festgelegt. Mit Hilfe der aufgezeigten Parameter kann über einstellbare Größen das gewünschte Eigenschaftsprofil der Beschichtung, abgestimmt auf die verschiedenen Trägermaterialien und Anwendungen, sehr genau eingestellt werden.

Das Trägermaterial wird bevorzugt mit einer Geschwindigkeit von größer 2 m/min, bevorzugt 20 bis 100 m/min, beschichtet, wobei die Beschichtungstemperatur größer als die Erweichungstemperatur zu wählen ist.

### Beispiel 1

Elastische medizinische Binden wurden direkt beschichtet.

Durch die offenbarte Erfindung konnten der Hilfsträger für die indirekte Beschichtung sowie die umweltgerechte Rückgewinnung des Lösemittels, welche kostenintensiv ist und einen hohen Maschinenaufwand zur Folge hat, eingespart werden. Die Binde wurde im Thermosiebdruck mit einem Masseauftrag von 160 g/m² Klebemasse auf Basis eines Blockcopolymeren beschichtet.
Bei dem Blockcopolymeren handelte es sich um ein Styrol-Ethylen-Butylen-Styrol-Blockcopolymerisat, welches mit Paraffinkohlenwasserstoffewachsen versetzt worden ist. Das Verhältnis war ein Teil Polymer auf ein Teil Paraffinkohlenwasserstoff. Zu dieser Mischung wurden 10 % Polystyrolharz (Amoco 18240) gegeben. Der Kleber enthielt ein Prozent Irganox, ein Alterungsschutzmittel (β-(3,5 Di-t.butyl-4-hydroxyphenyl)-propionsäure-n-octadecylester)), und weitere Kohlenwasserstoffharze und Fettsäureester, welche im Gesamtkleber nur zu geringen Mengen enthalten waren. Der Erweichungspunkt dieser Klebemasse betrug ca. 100 °C (DIN 52011) und die Glasübergangstemperatur, bestimmt nach oben genannter Methode, -6 °C.

Folgende Kennwerte der elastischen und plastischen Eigenschaften der Binde wurden gemessen:

| | herkömmliche direkte Thermosiebdruckbeschichtung | erfindungsgemäße direkte Thermosiebdruckbeschichtung |
|---|---|---|
| Dehnung bei 10 N/cm | 45 % | 87 % |
| Plastische Verformung bei 10 N/cm | 25 % | 25 % |

Mit einer 14 Mesh Siebschablone konnte der hohe Masseauftrag erzielt werden. Durch die Verwendung der großen Beschichtungspunkte konnte eine gute Haftung auf dem Träger und ein sauberes Schneiden erzielt werden. Erfindungsgemäß war die Klebemasse hautverträglich und gut haut- und trägerrückseitenverklebend.

Als adhäsive Beschichtung wurde eine 1,8 %ige thermisch vernetzende Silikonlösung eingesetzt. Die Reaktionszeit betrug 10 Minuten bei 120° C; als Beschichtungsverfahren wurde das Sprühverfahren gewählt.

Die so hergestellte Binde war auch in einem mehrlagigen Verband luftdurchlässig (mehr als 15 cm³/(cm²*s) und wasserdampfdurchlässig (mehr als 1500 g/(m²*24h).

Die elastische adhäsive Binde wurde für Kompressions- Stütz und Entlastungsverbände eingesetzt, wobei die hohe Sofort-, Dauerklebkraft und Scherfestigkeit vorteilhaft waren. Die Modellierbarkeit und Anwenderempfindung waren durch die den partiellen Auftrag der Klebemasse verbessert worden.

## Patentansprüche

1. Verfahren zur zumindest partiellen direkten Beschichtung eines dehnfähigen Trägermaterials mit einer Haftklebemasse, wobei das Trägermaterial mittels einer transportierenden Vorrichtung derartig gegen eine Beschichtungsvorrichtung geführt wird, daß durch die Beschichtungsvorrichtung die Haftklebemasse auf das Trägermaterial aufgetragen wird, **dadurch gekennzeichnet, daß**
die Beschichtungsvorrichtung abhäsiv ausgerüstet ist.

2. Verfahren nach Anspruch 1, **dadurch gekennzeichnet, daß** die Beschichtung des Trägermaterials vollflächig erfolgt.

3. Verfahren nach Anspruch 1, **dadurch gekennzeichnet, daß**
die abhäsive Ausrüstung aus Silikonen oder Fluor enthaltenden Verbindungen oder plasmabeschichtete Trennsysteme besteht.

4. Verfahren nach Anspruch 1, **dadurch gekennzeichnet, daß**
der abhäsive Auftrag mit einem Flächengewicht von 0,01 g/m² bis 350 g/m² aufgetragen ist, bevorzugt 0,1 g/m² bis 10 g/m².

5. Verfahren nach Anspruch 1, **dadurch gekennzeichnet, daß**
die transportierenden Vorrichtung besteht aus einer Druckwalze und die Beschichtungsvorrichtung aus einer rotierenden, nahtlosen, trommelförmigen und perforierten sowie abhäsiv ausgerüsteten Rundschablone, die über Düse mit der Haftklebemasse beschickt wird, wobei die Haftklebemasse über eine Düsenlippe durch die Rundschablone auf das vorbeigeführte Trägermaterial aufgebracht wird.

6. Verfahren nach Anspruch 1, **dadurch gekennzeichnet, daß**
auf der transportierenden Vorrichtung Haft- oder Halteeinrichtungen vorhanden sind, so daß bei der Beschichtung die Eigenschaften des Trägers nicht verändert werde

7. Verfahren nach Anspruch 6, **dadurch gekennzeichnet, daß**
die Haltevorrichtungen aus Nadeln bestehen, die aus der transportierenden Vorrichtung ragen und in das Trägermaterial eingreifen.

8. Verfahren nach Anspruch 7, **dadurch gekennzeichnet, daß**
die Nadeln eine Länge aufweisen größer 10 µm, bevorzugt zwischen 30 µm und 5000 µm, besonders bevorzugt zwischen 35 µm und 1000 µm.

9. Verfahren nach Anspruch 7, **dadurch gekennzeichnet, daß**
die Nadeln zumindest teilweise auf der Oberfläche der transportierenden Vorrichtung beweglich sind.

10. Verfahren nach Anspruch 6, **dadurch gekennzeichnet, daß**
die Haltevorrichtungen aus einer aufgerauhten Oberfläche bestehen, deren Rauhigkeitsgeometrien in das Trägermaterial eingreifen.

11. Verfahren nach Anspruch 10, **dadurch gekennzeichnet, daß** die Rauhtiefe der Oberfläche zwischen 30 und 5000 µm liegt.

12. Verfahren nach Anspruch 10, **dadurch gekennzeichnet, daß** die aufgerauhte Oberfläche besteht aus aufgebrachten Hartstoffpartikeln und/oder einer durch das Formgebungsverfahren oder durch mechanische, physikalische oder chemische Behandlung aufgerauhten Metall-, Keramik- oder Kunststoffoberfläche.

13. Verfahren nach Anspruch 6, **dadurch gekennzeichnet, daß**
die Haltevorrichtung aus einer selbsthaftenden Oberfläche besteht, deren Adhäsionskräfte auf das Trägermaterial wirken.

14. Verfahren nach Anspruch 6, **dadurch gekennzeichnet, daß**
die Haltevorrichtungen aus elektromagnetisch Feldern besteht, deren Kräfte auf das Trägermaterial wirken.

## Claims

1. Process for the at least partial, direct coating of an extensible backing material with a pressure-sensitive adhesive composition, the backing material being guided by a transporting apparatus against a coating apparatus in such a way that the latter applies the pressure-sensitive adhesive composition to the backing material, **characterized in that** the coating apparatus has received an anti-adhesive treatment.

2. Process according to Claim 1, **characterized in that** the backing material is coated over its whole area.

3. Process according to Claim 1, **characterized in that** the anti-adhesive treatment consists of silicones or fluorine compounds or plasma-coated release systems.

4. Process according to Claim 1, **characterized in that** the anti-adhesive layer has a weight per unit area of from 0.01 to 350 g/m², preferably 0.1 to 10 g/m².

5. Process according to Claim 1, **characterized in that** the transporting apparatus consists of a printing roller and the coating apparatus of a rotating, seamless, drum-shaped and perforated and anti-adhesively treated cylindrical screen which is fed via a nozzle with the pressure-sensitive hotmelt adhesive composition, the pressure-sensitive adhesive composition being applied by way of a nozzle lip through the cylindrical screen and onto the backing material that is conveyed past it.

6. Process according to Claim 1, **characterized in that** there are adhesion devices or holding devices present on the transporting apparatus so that the properties of the backing are not altered in the course of coating.

7. Process according to Claim 6, **characterized in that** the holding apparatuses consist of needles which project from the transporting apparatus and engage in the backing material.

8. Process according to Claim 7, **characterized in that** the needles have a length greater than 10 µm, preferably between 30 and 5000 µm and, with particular preference, between 35 and 1000 µm.

9. Process according to Claim 7, **characterized in that** the needles are at least in part mobile on the surface of the transporting apparatus.

10. Process according to Claim 6, **characterized in that** the holding apparatuses consist of a roughened surface whose roughness geometries engage in the backing material.

11. Process according to Claim 10, **characterized in that** the peak-to-valley roughness of the surface is between 30 and 5000 µm.

12. Process according to Claim 10, **characterized in that** the roughened surface consists of applied particles of hard material and/or of a metal, ceramic or plastic surface roughened by the shaping procedure or by means of mechanical, physical or chemical treatment.

13. Process according to Claim 6, **characterized in that** the holding apparatus consists of a self-adhesive surface whose forces of adhesion act on the backing material.

14. Process according to Claim 6, **characterized in that** the holding apparatuses consist of electromagnetic fields whose forces act on the backing material.

## Revendications

1. Procédé de revêtement direct au moins partiel d'un matériau de support extensible par une pâte adhésive, le matériau de support étant guidé contre un dispositif de revêtement au moyen d'un dispositif de transport de telle sorte que la pâte adhésive soit appliquée sur le matériau de support par le dispositif de revêtement, **caractérisé en ce que** le dispositif de revêtement est rendu non adhérent.

2. Procédé selon la revendication 1, **caractérisé en ce que** le revêtement du matériau de support s'effectue sur toute sa surface.

3. Procédé selon la revendication 1, **caractérisé en ce que** le revêtement non adhérent est constitué de silicones, de composés contenant du fluor ou de systèmes de séparation appliqués par plasma.

4. Procédé selon la revendication 1, **caractérisé en ce que** le revêtement non adhérent est appliqué à une masse par unité de surface de 0,01 g/m² à 350 g/m² et de préférence de 0,1 g/m² à 10 g/m².

5. Procédé selon la revendication 1, **caractérisé en ce que** le dispositif de transport est constitué d'un cylindre d'impression et le dispositif de revêtement d'un pochoir rond rotatif, sans soudure, en forme de tambour perforé et rendu non adhérent qui reçoit la pâte adhésive par des ajutages, la pâte adhésive étant appliquée par une lèvre d'ajutage et à travers le pochoir rond sur le matériau de support qui est guidé devant elle.

6. Procédé selon la revendication 1, **caractérisé en ce que** sur le dispositif de transport sont prévus des dispositifs d'adhérence ou de maintien qui ne modifient pas les propriétés du support lors du revêtement.

7. Procédé selon la revendication 6, **caractérisé en ce que** les dispositifs de maintien sont constitués d'aiguilles qui débordent hors du dispositif de transport et qui s'engagent dans le matériau de support.

8. Procédé selon la revendication 7, **caractérisé en ce que** les aiguilles ont une longueur supérieure à 10 µm, de préférence comprise entre 30 µm et 5000 µm et de façon particulièrement préférable entre 35 µm et 1000 µm.

9. Procédé selon la revendication 7, **caractérisé en ce que** sur au moins une partie de la surface du dispositif de transport, les aiguilles sont mobiles.

10. Procédé selon la revendication 6, **caractérisé en ce que** les dispositifs de maintien sont constitués d'une surface rugueuse dont la géométrie des rugosités s'engage dans le matériau de support.

11. Procédé selon la revendication 10, **caractérisé en ce que** la profondeur des rugosités de la surface est comprise entre 30 et 5000 µm.

12. Procédé selon la revendication 10, **caractérisé en ce que** la surface rugueuse est constituée de particules de matière dure qui y sont appliquées et/ou d'une surface métallique, céramique ou synthétique rendue rugueuse par un traitement mécanique, physique ou chimique.

13. Procédé selon la revendication 6, **caractérisé en ce que** le dispositif de maintien est constitué d'une surface autocollante dont les forces d'adhérence agissent sur le matériau de support.

14. Procédé selon la revendication 6, **caractérisé en ce que** les dispositifs de maintien sont constitués de champs électromagnétiques dont les forces agissent sur le matériau de support.
